(19) **Europäisches Patentamt / European Patent Office / Office européen des brevets**

(11) **EP 3 427 649 B1**

(12) **EUROPEAN PATENT SPECIFICATION**

(45) Date of publication and mention
of the grant of the patent:
**10.03.2021 Bulletin 2021/10**

(51) Int Cl.:
*A61B 5/021* *(2006.01)* *A61B 5/029* *(2006.01)*
*A61B 5/08* *(2006.01)* *A61B 5/00* *(2006.01)*
*A61B 5/022* *(2006.01)* *A61B 5/11* *(2006.01)*
*A61B 5/145* *(2006.01)*

(21) Application number: **17782508.0**

(22) Date of filing: **14.04.2017**

(86) International application number:
**PCT/JP2017/015276**

(87) International publication number:
**WO 2017/179695 (19.10.2017 Gazette 2017/42)**

(54) **BIOLOGICAL INFORMATION ANALYZING DEVICE, SYSTEM, AND PROGRAM**

VORRICHTUNG, SYSTEM UND PROGRAMM ZUR ANALYSE BIOLOGISCHER INFORMATIONEN

DISPOSITIF, SYSTÈME ET PROGRAMME D'ANALYSE D'INFORMATIONS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2016 JP 2016082463**

(43) Date of publication of application:
**16.01.2019 Bulletin 2019/03**

(73) Proprietors:
- **Omron Corporation**
  **Kyoto-shi, Kyoto 600-8530 (JP)**
- **Omron Healthcare Co., Ltd.**
  **Muko-shi, Kyoto 617-0002 (JP)**

(72) Inventors:
- **NAKAJIMA, Hiroshi**
  **Kyoto-shi**
  **Kyoto 600-8530 (JP)**
- **WADA, Hirotaka**
  **Kyoto-shi**
  **Kyoto 600-8530 (JP)**
- **TSUCHIYA, Naoki**
  **Kyoto-shi**
  **Kyoto 600-8530 (JP)**
- **KASAI, Masaaki**
  **Kyoto-shi**
  **Kyoto 600-8530 (JP)**
- **KAN, Eriko**
  **Kyoto-shi**
  **Kyoto 600-8530 (JP)**

- **UENOYAMA, Toru**
  **Tokyo 108-0075 (JP)**
- **OBAYASHI, Keiichi**
  **Kyoto-shi**
  **Kyoto 600-8530 (JP)**
- **KOKUBO, Ayako**
  **Kyoto-shi**
  **Kyoto 600-8530 (JP)**
- **OTA, Yuya**
  **Kyoto-shi**
  **Kyoto 600-8530 (JP)**
- **SHIGA, Toshikazu**
  **Muko-shi**
  **Kyoto 617-0002 (JP)**
- **KUWABARA, Mitsuo**
  **Muko-shi**
  **Kyoto 617-0002 (JP)**
- **SATO, Hironori**
  **Muko-shi**
  **Kyoto 617-0002 (JP)**
- **MIYAGAWA, Ken**
  **Muko-shi**
  **Kyoto 617-0002 (JP)**
- **TSUTSUMI, Masakazu**
  **Muko-shi**
  **Kyoto 617-0002 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(56) References cited:
EP-A1- 1 334 693      CN-A- 104 511 150
JP-A- H1 133 003      JP-A- H08 229 012
JP-A- H11 128 186     JP-A- 2003 024 310

JP-A- 2013 094 222    JP-A- 2013 208 140
JP-B2- 3 820 719

**Description**

TECHNICAL FIELD

The present invention relates to a technique for obtaining useful information from a measured blood pressure waveform.

RELATED ART

Techniques are known in which changes in the internal pressure of the radial artery are measured and the shape of pressure pulses (a blood pressure waveform) is recorded. Patent Document 1 (JP 2008-61824A) discloses measuring a blood pressure waveform through tonometry and obtaining information such as AI (Augmentation Index) value, pulse wave period, baseline fluctuation rate, sharpness, ET (Ejection Time), and the like from the blood pressure waveform. Patent Document 2 (JP 2005-532111A), meanwhile, discloses measuring a blood pressure waveform using a wristwatch-type blood pressure monitor, calculating a mean arterial pressure, a mean systolic pressure, a mean diastolic pressure, a mean systolic pressure index, and a mean diastolic pressure index from the blood pressure waveform, and then outputting an alert when those values deviate from reference values.

RELATED ART DOCUMENTS

PATENT DOCUMENTS

Patent Document 1: JP 2008-61824A
Patent Document 2: JP 2005-532111A

EP 1 334 693 A1 describes an exercise intensity measuring device.
JP 3 820719 B2 describes a biological-condition measuring device in order to measure the state of the human body as living organism.

SUMMARY OF THE INVENTION

PROBLEM TO BE SOLVED BY THE INVENTION

The inventors of the present invention are undertaking diligent research toward putting into practical use blood pressure measurement devices capable of accurately measuring a blood pressure waveform for each heartbeat during free movement. Through experiments using test subjects throughout the course of this development, the inventors of the present invention discovered that many types of useful information can be extracted from blood pressure waveform data measured continuously during free movement.
AI value is one indicator expressing the load on a person's heart. To find an AI value, it has been necessary to measure a blood pressure waveform for each heartbeat, and to do so, it has thus far been necessary to take the measurement during a state of rest. In other words, it has thus far not been possible to evaluate the load placed on a user's heart by the user's activity.
The inventors of the present invention discovered that the effects of user activity appear as changes in the shape of a blood pressure waveform measured continuously during free movement, and that the load such activity places on the heart can be evaluated from those changes.
An object of the present invention is to provide a novel technique for evaluating a load placed on a heart by activity.

MEANS FOR SOLVING THE PROBLEMS

To achieve the above-described object, the present invention employs the following configurations and refers to a biological information analyzing device as claimed in claim 1 and to a system according to claim 11, and to a program according to claim 12 and to a biological information analyzing method according to claim 13.
A biological information analyzing device according to the present invention is a biological information analyzing device including: an indicator extraction unit configured to extract, from blood pressure waveform data measured continuously by a sensor that is wearable on a user's body and can non-invasively measure a blood pressure waveform for each of a plurality of heartbeats, an indicator expressing a load placed on the user's heart by an activity; and a processing unit configured to output the indicator extracted by the indicator extraction unit, wherein the indicator extraction unit is configured to find the indicator on the basis of a difference between a blood pressure waveform in a resting section corresponding to when the user is in a resting state and a blood pressure waveform in an active section corresponding to when the user is engaged in the activity.

Here, the resting section is a section in which the user is at rest, and the active section is a section in which the user is active. The resting section and the active section may be distinguished by being input (taught) by a person (the user him/herself or a third party). For example, the indicator extraction unit may be configured to determine the active section and the resting section on the basis of data expressing an activity start time and data expressing an activity end time for the user. In this case, the indicator extraction unit can determine that the section from the activity start time to the activity end time is the active section, and that other sections are resting sections. The resting section and the active section may be distinguished by the biological information analyzing device automatically recognizing those sections on the basis of measurement data. For example, the indicator extraction unit may be configured to determine the active section and the resting section on the basis of measurement data from a second sensor (a body movement measurement sensor, an environment measurement sensor, or the like) that detects user activity.

The indicator extraction unit is configured to obtain a time at which the effect of user activity appears as a change in the blood pressure waveform (called an "effect appearance point"), and to find an indicator on the basis of the amount of time that has passed from the start of the activity to the effect appearance point. The indicator may be the amount of time that has passed itself, or may be a value determined on the basis of the amount of time that has passed. A shorter amount of time that has passed can be said to indicate a greater load placed on the heart by the activity. The indicator extraction unit may be configured to find the first point, among ejection start points following the start of the activity, satisfying a condition in which a difference between a blood pressure value at that point in time and a representative minimum blood pressure value in the resting section is greater than or equal to a threshold, as the effect appearance point. The representative minimum blood pressure value in the resting section may be, for example, the minimum blood pressure value for one beat immediately before the active section, or the mean value of the minimum blood pressure value for a predetermined number of beats immediately before the active section.

The indicator extraction unit may be configured to calculate the indicator on the basis of a difference between a representative minimum blood pressure value in the resting section and a representative minimum blood pressure value in the active section. The indicator may be the difference itself, or may be a value determined on the basis of the difference. The representative minimum blood pressure value in the active section may, for example, be a mean minimum blood pressure value in the active section or a maximum value of the minimum blood pressure value in the active section. Alternatively, the representative minimum blood pressure value in the resting section may, for example, be a mean minimum blood pressure value from a predetermined amount of time immediately before the active section. A greater difference between these minimum blood pressure values can be said to indicate a greater load placed on the heart by the activity.

The indicator extraction unit may also be configured to calculate an indicator on the basis of a degree of similarity between the shapes of the blood pressure waveform in the resting section and the blood pressure waveform in the active section. The indicator may be the shape similarity itself, or may be a value determined on the basis of the shape similarity. The shape similarity may be found as a correlation (e.g., cross-correlation) between a representative blood pressure waveform in the resting section and a representative blood pressure waveform in the active section. The representative blood pressure waveform in the active section may, for example, be a blood pressure waveform of one beat or a mean blood pressure waveform of several beats following an effect appearance point that is a time at which an effect of the activity appears as a change in the blood pressure waveform. The representative blood pressure waveform in the resting section may, for example, be a blood pressure waveform of one beat or a mean blood pressure waveform of several beats immediately before the active section. A lower blood pressure waveform shape similarity between the resting section and the active section, i.e., a greater change in the blood pressure waveform caused by the activity, can be said to indicate a greater load placed on the heart by the activity.

Additionally, the indicator extraction unit may be configured to find a plurality of sub-indicators on the basis of a difference between the blood pressure waveform in the resting section and the blood pressure waveform in the active section, and to calculate the indicator (an overall indicator) on the basis of the plurality of sub-indicators. Here, the amount of time that has passed from the start of the activity to the effect appearance point, the difference in the representative minimum blood pressure values between the resting section and the active section, and the shape similarity of representative blood pressure waveforms in the resting section and the active section can be given as examples of the sub-indicators. The overall indicator may also be determined in light of an AI value.

If the overall indicator is calculated on the basis of a plurality of sub-indicators as described above, the processing unit may be configured to output the overall indicator along with the sub-indicators. At least one of a graph format and a table format can be employed as the output format. The graph may output the overall indicator with at least one of the sub-indicators serving as a coordinate axis. In the graph, the overall indicator may be plotted at a size based on the value, or may be plotted along with the value, so that the value of the overall indicator can be understood.

The user's activity may be determined on the basis of data input by a person (the user him/herself or a third party), or may be determined on the basis of measurement data from a sensor that detects the user's activity.

A biological information analyzing system according to the present invention is a biological information analyzing system including: a sensor, which is worn on a user's body and can non-invasively measure a blood pressure waveform for each of heartbeats; and a biological information analyzing device as described above configured to analyze biological information using data of the blood pressure waveform measured continuously by the sensor.

A program according to the present invention is a program causing a processor to function as the indicator extraction unit and the processing unit of the biological information analyzing device.

The present invention can also be realized as a biological information analyzing method : as defined in claim 13.

EFFECTS OF THE INVENTION

According to the present invention, a technique for evaluating a load placed on a heart by activity can be provided.

BRIEF DESCRIPTION OF THE DRAWINGS

Fig. 1 is a diagram illustrating the external appearance of the overall configuration of a biological information analyzing system 10.

Fig. 2 is a block diagram illustrating the hardware configuration of the biological information analyzing system 10.

Fig. 3 is a cross-sectional view schematically illustrating the structure of a blood pressure measurement unit 20 and a measurement state.

Fig. 4 is a diagram illustrating a blood pressure waveform measured by the blood pressure measurement unit 20.

Fig. 5 is a block diagram illustrating processing performed by a biological information analyzing device 1.

Fig. 6 is a diagram illustrating the waveform of a pressure pulse (a blood pressure waveform) in the radial artery, for a single heartbeat.

Fig. 7 is a diagram illustrating an example of a blood pressure waveform obtained when a user spreads his/her legs while laying down (face-up), according to an Example.

Fig. 8 is a diagram illustrating indicators according to an Example.

Figs. 9A and 9B are diagrams illustrating an example of a screen display according to an Example.

Fig. 10 is a flowchart illustrating the flow of a process according to an Example.

EMBODIMENTS OF THE INVENTION

A preferred embodiment of the present invention will be described below with reference to the drawings. Note, however, that the descriptions of configurations given hereinafter should be changed as appropriate depending on the configuration of the device to which the invention is applied, various types of conditions, and so on, and the scope of the invention is not intended to be limited by the following descriptions.

Biological Information Analyzing System

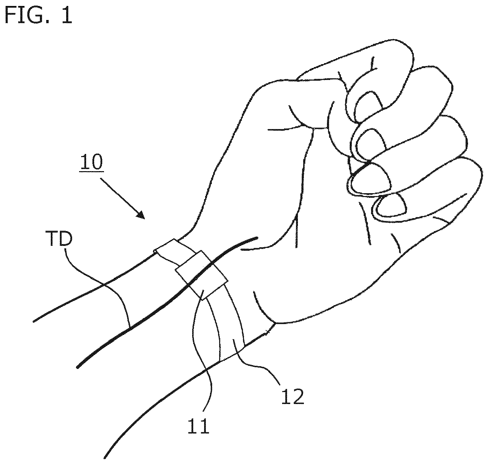

Fig. 1 is a diagram illustrating the external appearance of the overall configuration of a biological information analyzing system 10 according to an embodiment of the present invention. Fig. 1 illustrates a state in which the biological information analyzing system 10 is worn on the left wrist. The biological information analyzing system 10 includes a main unit 11 and a belt 12 fixed to the main unit 11. The biological information analyzing system 10 is what is known as a wearable device, and is worn so that the main unit 11 is in contact with the skin on the inner side of the wrist and so that the main unit 11 is arranged above a radial artery TD located beneath the skin. Although the present embodiment describes a configuration in which the device is worn above the radial artery TD, the configuration may be such that the device is worn above another superficial artery.

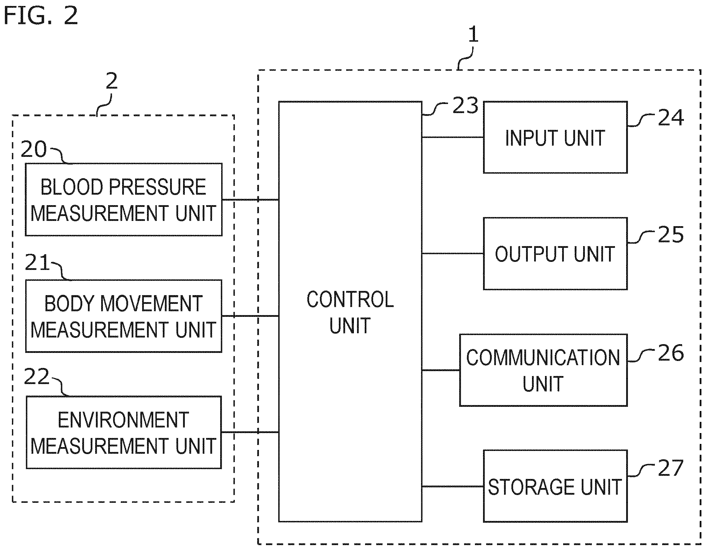

Fig. 2 is a block diagram illustrating the hardware configuration of the biological information analyzing system 10. Broadly speaking, the biological information analyzing system 10 includes a measurement unit 2 and a biological information analyzing device 1. The measurement unit 2 is a device that obtains information used to analyze biological information through measurement, and includes a blood pressure measurement unit 20, a body movement measurement unit 21, and an environment measurement unit 22. However, the configuration of the measurement unit 2 is not limited to that illustrated in Fig. 2. For example, units that measure biological information aside from blood pressure and body movement (body temperature, blood sugar, brain waves, and so on) may be added. Alternatively, units not used in the Example described later are not required configurations and may therefore be omitted from the biological information analyzing system 10. The biological information analyzing device 1 is a device that analyzes biological information on the basis of information obtained from the measurement unit 2, and includes a control unit 23, an input unit 24, an output unit 25, a communication unit 26, and a storage unit 27. The units 20 to 27 are connected to each other by a local bus and other signal lines so as to be capable of exchanging signals. The biological information analyzing system 10 also includes a power source (a battery), which is not illustrated.

The blood pressure measurement unit 20 is a unit that measures pressure pulses in the radial artery TD through tonometry. Tonometry is a non-invasive method of measuring pressure pulses using a pressure sensor, in which an artery is compressed from above the skin at an appropriate pressure to form a flat part in an artery TD, and the internal pressure and external pressure of the artery are balanced.

The body movement measurement unit 21 is a unit, including a three-axis accelerometer, that measures movement of a user's body (body movement) using the accelerometer. The body movement measurement unit 21 may include a circuit that converts the output of the three-axis accelerometer into a format that can be read by the control unit 23.

The environment measurement unit 22 is a unit that measures environment information that can affect the user's physical/mental state (and blood pressure in particular). The environment measurement unit 22 can include a temperature sensor, a humidity sensor, an illuminance sensor, an altitude sensor, a location sensor, and the like, for example. The environment measurement unit 22 may include a circuit that converts the output of these sensors into a format that can be read by the control unit 23.

The control unit 23 is a unit that handles a variety of processes, such as controlling the various parts of the biological information analyzing system 10, acquiring data from the measurement unit 2, storing the acquired data in the storage unit 27, processing/analyzing the data, inputting/outputting the data, and so on. The control unit 23 includes a hardware processor (called a CPU hereinafter), ROM (Read-Only Memory), RAM (Random Access Memory), and the like. The processing carried out by the control unit 23, which will be described later, is realized by the CPU reading programs stored in the ROM or the storage unit 27 and executing those programs. The RAM functions as a work memory when the control unit 23 carries out various types of processes. Although the present embodiment describes a configuration in which the control unit 23 acquires the data from the measurement unit 2 and stores the data in the storage unit 27, the configuration may be such that the data is stored (written) directly from the measurement unit 2 into the storage unit 27.

The constituent elements of the embodiment, e.g., the measurement units, an indicator extraction unit, a processing unit, a determination unit, a risk database, the input unit, the output unit, a case database, and the like may be provided as hardware in the biological information analyzing system 10. The indicator extraction unit, the processing unit, and the determination unit may receive executable programs stored in the storage unit 27 and execute those programs. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as necessary. Databases such as the risk database and the case database may be provided in the storage unit 27 or the like, and may store information arranged so that the data can be searched and accumulated with ease. The structure, operations, and the like of the biological information analyzing system 10 are disclosed in JP 2016-082069, for example.

The structure, operations, and the like of the blood pressure measurement unit are disclosed in JP 2016-087003A.

The input unit 24 is a unit that provides an operation interface to the user. For example, operation buttons, switches, a touch panel, or the like can be used.

The output unit 25 is a unit that provides, to the user, an interface that outputs information. For example, a display device that outputs information as images (a liquid crystal display or the like), an audio output device or a buzzer that outputs information as audio, an LED that outputs information by emitting or extinguishing light, a vibration device that outputs information as vibrations, or the like can be used.

The communication unit 26 is a unit that carries out data communication with other devices. Any system, including wireless LAN, Bluetooth (registered trademark), or the like, may be used as the data communication system.

The storage unit 27 is a storage medium in which data can be stored and from which data can be read out, and stores programs executed by the control unit 23, measurement data obtained from the measurement units, various types of data obtained by processing the measurement data, and so on. The storage unit 27 is a medium that electrically, magnetically, optically, mechanically, or chemically stores the information to be stored. Flash memory can be used, for example. The storage unit 27 may be a portable type such as a memory card, or may be built into the biological information analyzing system 10.

Some or all of the body movement measurement unit 21, the environment measurement unit 22, the control unit 23, the input unit 24, the output unit 25, and the storage unit 27 may be configured as devices separate from the main unit 11. In other words, as long as the main unit 11 including the blood pressure measurement unit 20 and a circuit that controls the blood pressure measurement unit 20 can be worn on a wrist, the structures of other units can be designed as desired. In this case, the main unit 11 is linked to the other units via the communication unit 26. A variety of configurations are conceivable, such as implementing the functions of the control unit 23, the input unit 24, the output unit 25, and so on as a smartphone app, or obtaining necessary data from an activity meter having the functions of the body movement measurement unit 21, the environment measurement unit 22, and so on. Sensors that measure biological information aside from blood pressure may be provided as well. For example, a sleep sensor, a pulse oximeter ($SpO_2$ sensor), a breathing sensor (flow sensor), a blood sugar level sensor, and so on may be combined as well.

Although the present embodiment describes providing a sensor that measures blood pressure (the blood pressure measurement unit 20) and a configuration that carries out analysis processes on blood pressure waveform data (the control unit 23 and the like) in a single device, these elements may be configured as separate entities. In the present embodiment, a configuration that carries out analysis processes on biological information (the control unit 23 and the like) is called a "biological information analyzing device", and a device configured by combining a measurement unit and the biological information analyzing device is called a "biological information analyzing system". However, these names are used for the sake of simplicity, and the measurement unit and the configuration that carries out analysis processes on biological information may as a whole be called a "biological information analyzing device", or other names may be used instead.

Blood Pressure Waveform Measurement

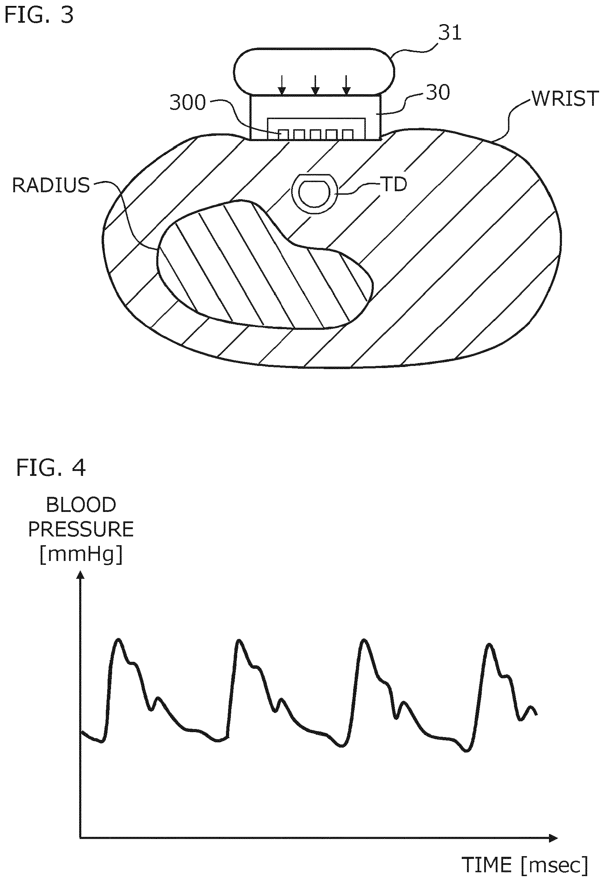

Fig. 3 is a cross-sectional view schematically illustrating the structure of the blood pressure measurement unit 20 and a measurement state. The blood pressure measurement unit 20 includes a pressure sensor 30, and a compression mechanism 31 for pressing the pressure sensor 30 against the wrist. The pressure sensor 30 includes a plurality of pressure detection elements 300. The pressure detection elements 300 are elements that detect a pressure and convert the pressure into an electrical signal, and elements that use a piezoresistance effect, for example, can be favorably used. The compression mechanism 31 is constituted by, for example, an air bladder and a pump that adjusts the internal pressure of the air bladder. When the control unit 23 controls the pump to increase the internal pressure of the air bladder, the air bladder expands and presses the pressure sensor 30 against the surface of the skin. Note that the compression mechanism 31 may be any mechanism capable of adjusting a compressive force of the pressure sensor 30 against the surface of the skin, and is not limited to a mechanism employing an air bladder.

When the biological information analyzing system 10 is secured to the wrist and started, the control unit 23 controls the compression mechanism 31 of the blood pressure measurement unit 20 to keep the compressive force of the pressure sensor 30 in an appropriate state (a tonometry state). Pressure signals detected by the pressure sensor 30 are then acquired sequentially by the control unit 23. The pressure signals obtained by the pressure sensor 30 are generated by taking analog physical amounts (e.g., voltage values) outputted by the pressure detection elements 300 and digitizing those physical amounts through an A/D conversion circuit or the like that employs a known technique. Suitable analog values such as current values, resistance values, or the like may be employed as the analog physical amounts, depending on the types of the pressure detection elements 300. The signal processing such as A/D conversion may be carried out by providing a predetermined circuit in the blood pressure measurement unit 20, or may be carried out by another unit (not shown) provided between the blood pressure measurement unit 20 and the control unit 23. The pressure signals acquired by the control unit 23 correspond to instantaneous values of the internal pressure of the radial artery TD. Accordingly, time series data of a blood pressure waveform can be obtained by acquiring a pressure signal at a time granularity and continuity that enable the blood pressure waveform of a single heartbeat to be obtained. The control unit 23 stores the pressure signals sequentially obtained from the pressure sensor 30 in the storage unit 27 along with information of the measurement times of the signals. The control unit 23 may store the acquired pressure signals as-is in the storage unit 27, or may store the pressure signals in the storage unit 27 after applying necessary signal processing to the pressure signals. The "necessary signal processing" may include processing for correcting the pressure signals so that the amplitude of the pressure signals matches a blood pressure value (e.g., an upper arm blood pressure), processing for reducing or removing noise from the pressure signals, or the like, for example.

Fig. 4 is a diagram illustrating a blood pressure waveform measured by the blood pressure measurement unit 20. The horizontal axis represents time, and the vertical axis represents blood pressure. The sampling frequency can be set as desired, but is preferably set to greater than or equal to 100 Hz in order to reproduce the shape characteristics of the waveform of a single heartbeat. Because the period of a single heartbeat is approximately one second, approximately 100 or more data points can be acquired in the waveform of a single heartbeat.

The blood pressure measurement unit 20 according to the present embodiment has advantages such as those described below.

A blood pressure waveform can be measured for each heartbeat. Thus for example, a variety of indicators related to blood pressure, heart condition, cardiovascular risk, and so on can be obtained on the basis of the shape characteristics of the blood pressure waveform. Additionally, the instantaneous value of the blood pressure can be monitored, which makes it possible to immediately detect blood pressure surges (sudden rises in blood pressure value), reliably detect blood pressure fluctuations and disturbances in the blood pressure waveform appearing only over extremely short amounts of time (one to several heartbeats), and so on.

Blood pressure monitors that are secured to the wrist or upper arm and measure blood pressure through the oscillometric method are in practical use as portable blood pressure monitors. However, a conventional portable blood pressure monitor can only measure a mean blood pressure value from fluctuations in the internal pressure of a cuff over several heartbeats, spanning several seconds to several tens of seconds, and thus cannot obtain time series data of a blood pressure waveform for each heartbeat, as is the case with the blood pressure measurement unit 20 according to the present embodiment.

The blood pressure waveform time series data can be recorded. When the blood pressure waveform time series data is acquired, a variety of indicators pertaining to blood pressure, heart condition, cardiovascular risk, and the like can be obtained by finding characteristics pertaining to changes in the blood pressure waveform over time, analyzing the frequency of the time series data and extracting specific frequency components, and so on, for example.

Because the device is configured as a portable (wearable) device, measurements place little burden on the user, and it is relatively easy to take continuous measurements for long periods of time, monitor blood pressure throughout the entire day, and so on. Furthermore, the portable form makes it possible not only to measure resting blood pressure, but also to measure changes in blood pressure during free movement (e.g., during daily activities, exercise, and so on). This in turn makes it possible to understand the effects of daily activities (sleeping, meals, commuting, work, taking medicine, and so on), exercise, and so on on blood pressure, for example.

Conventional products are devices of a type in which a blood pressure measurement unit is secured to the arm or wrist and the measurement is taken in a state of rest, and changes in blood pressure during daily activities, exercise, and so on cannot be measured, as with the biological information analyzing system 10 according to the present embodiment.

It is easy to link or combine the system with other sensors. For example, causal relationship evaluations or compound evaluations can be made using information obtained from other sensors (body movement, environment information such as temperature, other biological information such as $SpO_2$ or breathing, and the like).

Biological Information Analyzing Device

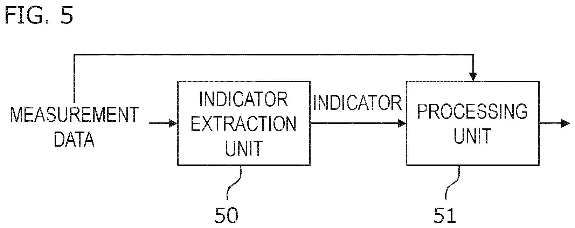

Fig. 5 is a block diagram illustrating processing performed by the biological information analyzing device 1. As illustrated in Fig. 5, the biological information analyzing device 1 includes an indicator extraction unit 50 and a processing unit 51. In the present embodiment, the processes of the indicator extraction unit 50 and the processing unit 51 may be realized by the control unit 23 executing necessary programs. These programs may be stored in the storage unit 27. When executing the necessary programs, the control unit 23 loads the programs in question, which are stored in the ROM or the storage unit 27, into the RAM. The control unit 23 then uses a CPU to interpret and execute the programs loaded into the RAM, and controls the various constituent elements. However, some or all the processing of the indicator extraction unit 50 and the processing unit 51 may be implemented by a circuit such as an ASIC, a FPGA, or the like. Alternatively, some or all of the processing of the indicator extraction unit 50 and the processing unit 51 may be implemented by a computer separate from the main unit 11 (e.g., a smartphone, a tablet terminal, a personal computer, a cloud server, or the like).

The indicator extraction unit 50 obtains, from the storage unit 27, the blood pressure waveform time series data measured continuously by the blood pressure measurement unit 20. The indicator extraction unit 50 extracts an indicator pertaining to characteristics of the blood pressure waveform, from the obtained blood pressure waveform time series data. Here, "characteristics of the blood pressure waveform" include shape characteristics of the blood pressure waveform for a single heartbeat, changes in the blood pressure waveform over time, a frequency component of the blood pressure waveform, and so on. The blood pressure waveform characteristics are not limited thereto, however. The extracted indicator is output to the processing unit 51. Because there are a variety of blood pressure waveform characteristics and indicators, the characteristics and indicators to be extracted can be designed and selected as appropriate in accordance with the purpose of the processing by the processing unit 51. The characteristics and indicators that can be extracted from the blood pressure waveform measurement data in the present embodiment will be described later.

When finding the indicator, the indicator extraction unit 50 can use the measurement data from the body movement measurement unit 21 and/or the measurement data from the environment measurement unit 22 in addition to the blood pressure waveform measurement data. Although not illustrated, measurement data from a sleep sensor, an $SpO_2$ sensor, a breathing sensor (flow sensor), a blood sugar level sensor, or the like may be combined as well. Carrying out a compound analysis on multiple types of measurement data obtained from multiple types of sensors enables a higher level of information analysis to be carried out on the blood pressure waveform. For example, the blood pressure waveform data can be classified into user states, such as resting and active, times of high and low temperature, and so on. Alternatively, causal relationships, correlations, and so on among the measurement data can be evaluated, by extracting the effects of body movement, activity amounts and activity intensity, changes in temperature, and so on on blood pressure.

The processing unit 51 receives the indicator extracted by the indicator extraction unit 50. The processing unit 51 carries out processing on the basis of the received indicator. A variety of processes are conceivable as the processes based on the indicator. For example, values of or changes in the extracted indicator may be provided to the user, a doctor, a nurse, or the like and used for health management, treatment, health guidance, and so on. Alternatively, circulatory system risk may be predicted, guidelines for maintaining one's health or reducing risks may be provided, or the like, on the basis of the extracted indicator. Furthermore, if a rise in cardiovascular risk has been detected or predicted on the basis of the indicator, the user or an attending doctor may be notified, control may be carried out to prevent activity that will place an excessive burden on the user's heart or prevent the occurrence of the circulatory system event, and so on.

Information Obtained from Blood Pressure Waveform

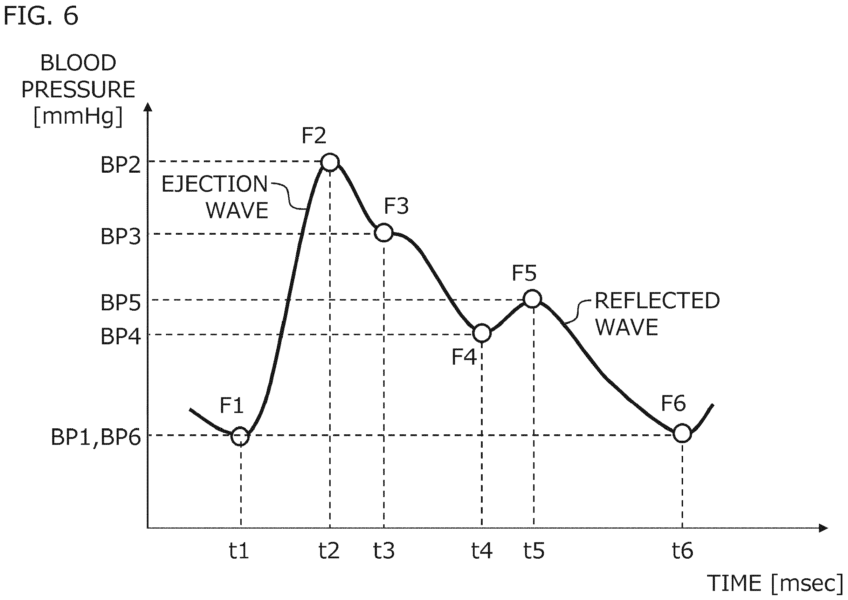

Fig. 6 is a diagram illustrating the waveform of a pressure pulse (a blood pressure waveform) in the radial artery, for a single heartbeat. The horizontal axis represents time t [msec], and the vertical axis represents blood pressure BP [mmHg].

The blood pressure waveform is a compound wave including an "ejection wave" produced when the heart contracts to expel blood and a "reflected wave" produced when the ejection wave is reflected by peripheral vessels, arterial branches, and so on. Examples of characteristic points that can be extracted from a blood pressure waveform corresponding to a single heartbeat are listed below.

· Point F1 is a point corresponding to the rise of the pressure pulse. The point F1 corresponds to an ejection start point of the heart, i.e., a point when the aortic valve opens.
· Point F2 is a point where the amplitude (pressure) of the ejection wave is maximum (a first peak).
· Point F3 is an inflection point appearing partway along the fall of the ejection wave due to the superposition of the reflected wave.
· Point F4 is a minimum point appearing between the ejection wave and the reflected wave, and is also called a "notch". This corresponds to a point when the aortic valve closes.
· Point F5 is a peak in the reflected wave appearing after point F4 (a second peak).
· Point F6 is the end point of the single heartbeat, and corresponds to the ejection start point of the next heartbeat, i.e., the starting point of the next heartbeat.

The indicator extraction unit 50 may use any algorithm to detect the characteristic points. For example, the indicator extraction unit 50 may extract a characteristic point (inflection point) of the blood pressure waveform by operating so as to find a nth-order differential waveforms of the blood pressure waveform and detect a zero crossing point thereof (for points F1, F2, F4, F5, and F6, this can be detected from a first-order differential waveform, and for point F3, from a second-order or fourth-order waveform). Alternatively, the indicator extraction unit 50 may identify the positions of the characteristic points by reading out a waveform pattern, in which characteristic points have been arranged in advance, from the storage unit 27, and fitting the blood pressure waveform in question to the waveform pattern.

By operating on the basis of the times t and the pressures BP of the above characteristic points F1 to F6, the indicator extraction unit 50 can obtain a variety of information (values, feature amounts, indicators, and the like) from the blood pressure waveform of a single heartbeat. The following provides representative examples of information that can be obtained from the blood pressure waveform. Note that tx and BPx represent the time and blood pressure, respectively, of a characteristic point Fx.

· pulse wave interval (heartbeat period) $TA = t6 - t1$
· heart rate $PR = 1/TA$
· pulse wave rise time $UT = t2 - t1$
· systole $TS = t4 - t1$
· diastole $TD = t6 - t4$
· reflected wave delay time $= t3 - t1$
· maximum blood pressure (systolic blood pressure) $SBP = BP2$
· minimum blood pressure (diastolic blood pressure) $DBP = BP1$
· mean blood pressure MAP = area of blood pressure waveform from t1 to t6 / heartbeat period TA
· systolic mean blood pressure = area of blood pressure waveform from t1 to t4 / systole TS
· diastolic mean blood pressure = area of blood pressure waveform from t4 to t6 / diastole TD
· pulse pressure PP = maximum blood pressure SBP - minimum blood pressure DBP
· late systolic pressure $SBP2 = BP3$
· AI (Augmentation Index) = (late systolic pressure SBP2 - minimum blood pressure DBP) / pulse pressure PP

Basic statistical amounts of this information (values, feature amounts, indicators) can also be used as indicators. The basic statistical amounts include representative values (mean values, median values, mode values, maximum values, minimum values, and the like) and dispersion (variance, standard deviation, coefficient of variation, and the like), for example. Changes over time in this information (values, characteristic values, indicators) can also be used as indicators.

By computing a plurality of pieces of beat information, the indicator extraction unit 50 can obtain an indicator called BRS (baroreflex sensitivity). This is an indicator expressing the capacity of blood pressure to regulate to a constant value. The spontaneous sequence method is an example of the calculation method. This is a method in which only a sequence in which the maximum blood pressure SBP and the pulse wave interval TA rise or fall in synchronization for three or more consecutive beats is extracted, the maximum blood pressure SBP and the pulse wave interval TA are plotted on a two-dimensional plane, and a slope obtained when a regression line is found through the least-squares method is defined as the BRS.

As described thus far, using the biological information analyzing system 10 according to the present embodiment makes it possible to obtain a variety of information from the blood pressure waveform data. However, the biological information analyzing system 10 need not include functions for obtaining all of the above-described information. It is acceptable to provide only the functions for obtaining the necessary information, in accordance with the configuration, user, purpose of usage, location of usage, and so on of the biological information analyzing system 10. Additionally, the configuration may be such that the functions are provided as program modules (application software), and functions can be added by installing the required program modules in the biological information analyzing system 10.

The following Examples describe specific examples of the application of the biological information analyzing system 10.

Example 1

The AI (Augmented Index) value is one indicator expressing the load on the heart, artery hardness, and so on. The AI value is found as an amplitude ratio between an ejection wave (drive pressure wave) produced by the ejection of blood from the left ventricle, and a reflected wave (reflected pressure wave) in which the ejection wave propagates through the circulatory system, is reflected by branches in the blood vessels, and returns. In other words,

$$\text{AI value} = (\text{late systolic pressure SBP2} - \text{diastolic blood pressure DBP}) / (\text{systolic blood pressure SBP} - \text{diastolic blood pressure DBP})$$

The higher the AI value is, the greater the afterload on the left ventricle is. It is thus thought that if the AI value can be measured while the user is active, the load placed on the left ventricle by that activity can be evaluated.

However, blood pressure waveform measurement devices according to conventional techniques can only measure blood pressure waveforms when the user is at rest (when seated, laying face-up, or the like). In other words, with blood pressure measurement devices according to conventional techniques, it is not possible to measure the AI value during activity and evaluate the load placed on the left ventricle by that activity.

In addition to when the user is resting, the above-described biological information analyzing system 10 can measure the blood pressure waveform during free movement as well. Indicators aside from the AI value can also be obtained. As such, this Example provides a biological information analyzing device capable of evaluating the load placed on the user's left ventricle by activity (exercise), on the basis of blood pressure waveform data obtained when the user is performing such activity.

Blood Pressure Waveform during Activity

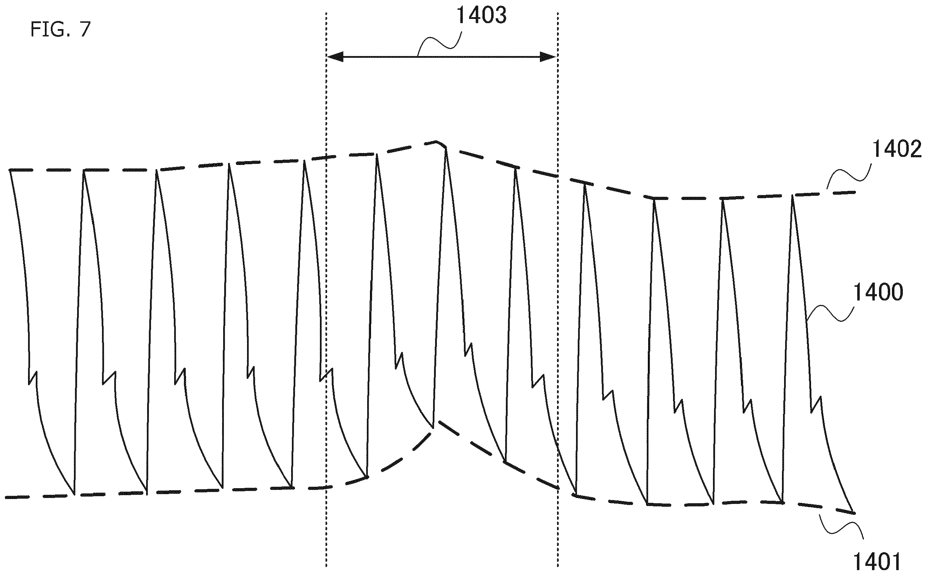

Fig. 7 illustrates a blood pressure waveform 1400 obtained when the user spreads his/her legs while laying down (laying face-up). The measurement was carried out by securing the biological information analyzing system 10 to the user's left wrist. A graph 1401 represents transitions in a minimum blood pressure (diastolic blood pressure) DBP, and a graph 1402 represents transitions in a maximum blood pressure value (systolic blood pressure value) SBP. The period 1403 in Fig. 7 represents a period in which the user spreads his/her legs (an active section), whereas in the other periods, the user is at rest (resting sections).

As illustrated in Fig. 7, it can be seen that user activity not only causes the AI value to change, but also produces a clear change in the blood pressure waveform. In other words, it is thought that an observable change in blood flow has also occurred in the left wrist to which the blood pressure measurement unit 20 is secured.

Indicators

Accordingly, in this Example, the effects of activity on the user's left ventricle are evaluated in light of the following indicators, in addition to the AI value.

1. Effect Appearance Time T

The first indicator is the time T from the start of user activity to when the effect appears in the blood pressure waveform (an effect appearance time).

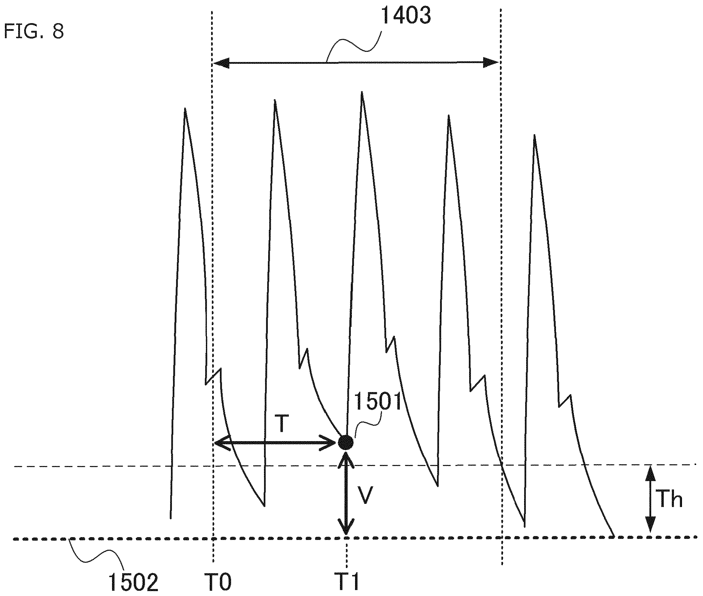

This will be described in detail with reference to Fig. 8. The effect appearance time T is the time from the user's activity start time T0 to a time T1 of an effect appearance point 1501 (T = T1 - T0). The effect appearance point 1501 is a point, among the ejection start points (minimum blood pressure) after the start of the user's activity, where the blood pressure value difference from a resting mean minimum blood pressure value (indicated by the dotted line 1502) is greater than or equal to a predetermined threshold Th. The resting mean minimum blood pressure value is an example of a representative minimum blood pressure value during rest, and can be found, for example, as the mean value of minimum blood pressure values from a predetermined amount of time proximate to the user's activity period 1403. It is thought that a lower effect appearance time T indicates a greater load on the left ventricle by the activity.

2. Minimum Blood Pressure Value Change Amount V

The second indicator is a minimum blood pressure value change amount V produced by user activity. In this Example, the change amount V is found as a difference between the blood pressure value at the effect appearance point 1501 and the resting mean minimum blood pressure value 1502. However, the change amount V is not limited to the difference between the blood pressure value at the effect appearance point 1501 and the resting mean minimum blood pressure value 1502, and may instead be found as a difference between a representative minimum blood pressure value in the resting section and a representative minimum blood pressure value in the active section. For example, the maximum value of the minimum blood pressure value in 1403 in the active section may be used as the representative minimum blood pressure value in the active section. It is thought that a greater change amount V indicates a greater load on the left ventricle by the activity.

3. Waveform Similarity S

The third indicator is a shape similarity S for the blood pressure waveform of a representative single beat at rest and during activity. The waveform similarity S can be found as a cross-correlation function between two representative waveforms, when at rest and during activity, normalized with respect to the time direction.

The blood pressure waveform of one beat immediately before the start of activity can be used as the representative waveform during rest. Alternatively, a mean blood pressure waveform of several beats immediately before the start of activity can be used as the representative waveform during rest. The blood pressure waveform of one beat starting from the effect appearance point 1501 can be used as the representative waveform during activity. Alternatively, a mean blood pressure waveform of several beats during the active section (e.g., a predetermined number of beats following the effect appearance point, all beats in which it is determined that the effect of activity is present, and so on) can be used as the representative waveform during activity.

A lower waveform similarity S indicates a greater change in the waveform due to activity, which is thought to indicate a greater load on the left ventricle by the activity.

Overall Indicator

In this Example, the indicator extraction unit 50 calculates an overall indicator E as follows, using the AI value and the above-described three indicators T, V, and S (all of which have been normalized).

$$E = AI + (1 - S) \times \{0.1 \times (1 - T) + 0.2V\}$$

Note that "AI" is the mean value of the AI values in the active section.

The definition of the overall indicator E given here is one example, and the overall indicator E may be defined so that the overall indicator E increases as the AI value increases, as the effect appearance time T decreases, as the change amount V increases, or as the waveform similarity S decreases. Additionally, not all of these indicators need be used.

Display Example

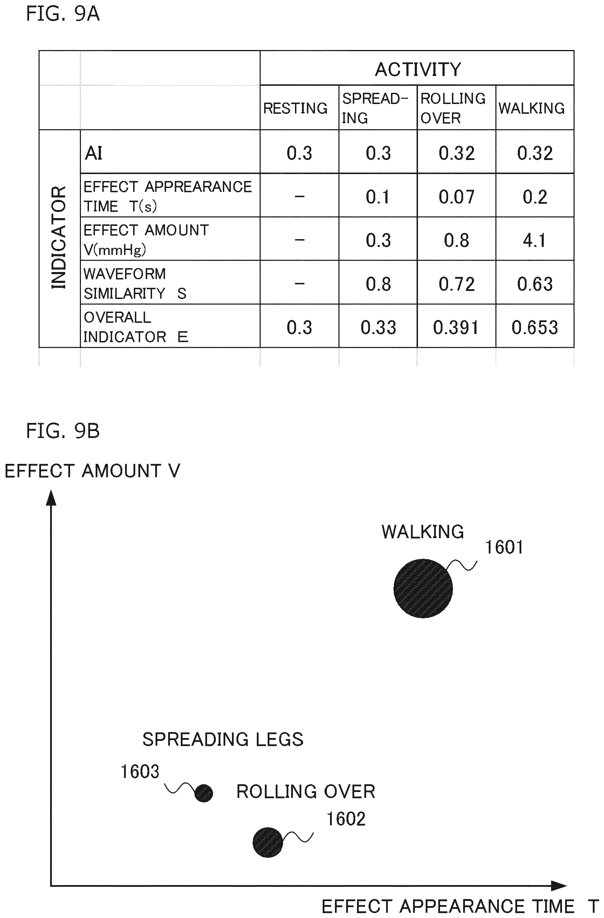

Fig. 9A is a diagram illustrating an example of a screen display made by the processing unit 51. In this example, various indicators are displayed in table format for activities including spreading the legs, rolling over, and walking. A display using such numerical values makes it easy to understand the extent of the effect each activity has on the left ventricle.

Fig. 9B is a diagram illustrating another example of a screen display made by the processing unit 51. In this example, graphs 1601, 1602, and 1603 for each of activities are drawn in positions that take the effect appearance time T and the change amount V as horizontal and vertical coordinates, and the areas of the graphs 1601, 1602, and 1603 express the magnitude of the overall indicator E. Although the effect appearance time T and the change amount V correspond to the horizontal and vertical axes in this example, combinations of other indicators may correspond to the horizontal and vertical axes, or three coordinates may be used to arrange the graphs three-dimensionally. By making a display using such graphs, whether a state is normal or abnormal can be determined with ease using not only the magnitude of the overall indicator E, but also the combinations of the indicators (T, V, S).

Processing Example

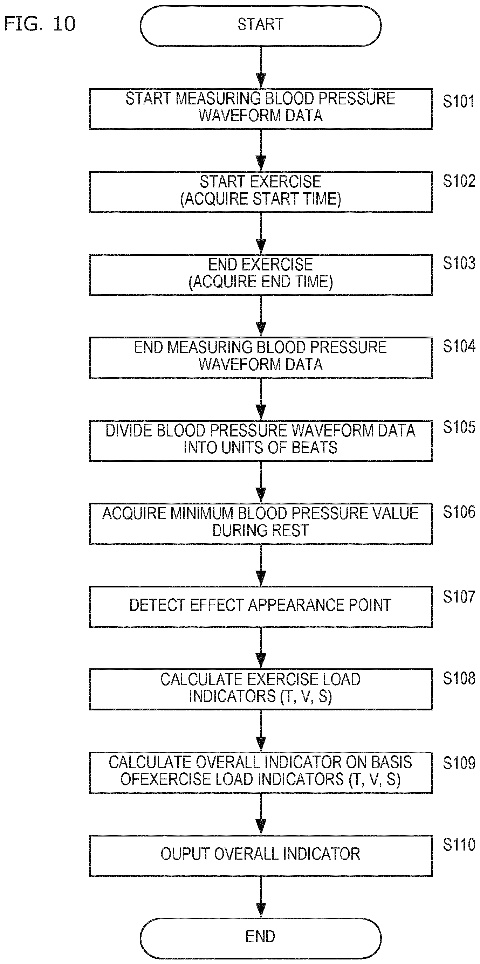

The flow of processing according to this Example will be described with reference to the flowchart in Fig. 10. In step S101, the biological information analyzing system 10 is secured to the user, and the measurement of the user's continuous blood pressure waveform data is started. After the start of measurement, a measurement is taken first in a resting state (seated, laying face-up, or the like). Next, in step S102, the user commences an activity (exercise) under the instruction of a doctor, rehabilitation instructor, or the like. In step S103, the user stops the activity (exercise) under the instruction of the instructor. The instructor inputs the exercise start time from step S102 and the exercise end time from step S103 into the biological information analyzing system 10 or another system as data. The data of the exercise start time and the exercise end time that has been input is stored in the storage unit 27 of the biological information analyzing system 10 or a storage unit in another system.

In step S104, the measurement of the user's continuous blood pressure waveform data by the biological information analyzing system 10 ends. The user's continuous blood pressure waveform data from between step S101 and S104 is stored in the storage unit 27 of the biological information analyzing system 10, as described above.

Note that it is not necessary for the user to exercise only once, and the exercise may be carried out several times by repeating the processing from steps S102 to S103. At this time, the type of exercise may vary. Additionally, the instructor may input the type of exercise instructed to the user into the biological information analyzing system 10 or the other system as data.

In step S105, the indicator extraction unit 50 acquires the blood pressure waveform time series data from the storage unit 27 and divides the data into units corresponding to beats. This division can be carried out by finding the ejection start point (point F1 in Fig. 6) from the blood pressure waveform data and taking the data from that point to the adjacent ejection start point as a single beat. The indicator extraction unit 50 stores a data index of the start point and end point of each beat in the storage unit 27. Because the end point of each beat matches the start point of the next beat, only a data index of the start points of the beats actually need be stored in the storage unit 27.

In step S106, the indicator extraction unit 50 finds a mean minimum blood pressure value during rest. Specifically, the indicator extraction unit 50 extracts the blood pressure waveform data from the resting section, obtains the minimum blood pressure value in each beat, and obtains the mean value of the obtained minimum blood pressure values. The resting section and the active section can be distinguished from each other on the basis of the data of the exercise start time and the exercise end time obtained from the storage unit 27 or another device. Beats present in both the resting section and the active section are excluded from the calculation of the mean minimum blood pressure value. Note that the indicator extraction unit 50 need not find the mean of the minimum blood pressure values for all of the beats in the resting section, and may instead find the minimum blood pressure values of a predetermined number of beats immediately before the activity is started (that is, the end of the resting section). The indicator extraction unit 50 stores the resting mean minimum blood pressure value that has been found in the storage unit 27.

In step S107, the indicator extraction unit 50 detects the effect appearance point after the start of exercise. As described above, the effect appearance point can be found as the start point of the first beat where the minimum blood pressure value is greater than the resting mean minimum blood pressure value by the threshold Th or more. Once the effect appearance point has been found, in step S108, the indicator extraction unit 50 finds the indicators T, V, S, and E using the effect appearance point.

The indicator T corresponds to the amount of time from the start of activity until the effect of activity appears in the blood pressure waveform, and thus the indicator extraction unit 50 can find the indicator T as the amount of time from the exercise start time (the start time of the active section) to the effect appearance point.

The indicator V is an amount of change in the minimum blood pressure value due to activity, and thus the indicator extraction unit 50 can find the indicator V as a difference between the resting mean minimum blood pressure value calculated in step S106 and the minimum blood pressure value in the beat at the effect appearance point. Note that the maximum value of the minimum blood pressure value in the active section, or the mean value of the minimum blood pressure values in a predetermined number of beats in the active section, may be used as the minimum blood pressure value during exercise.

The indicator S is the waveform similarity of the blood pressure waveforms in a single beat between during rest and during activity. The indicator extraction unit 50 first determines representative blood pressure waveforms during rest and during activity from the measurement data stored in the storage unit 27. A mean blood pressure waveform of several beats immediately before the start of activity can be used as the representative waveform during rest. The mean of the blood pressure waveform of one beat or a plurality of blood pressure waveforms starting from the effect appearance point can be used as the representative waveform during activity. The process for finding the mean of the blood pressure waveforms can be carried out by first carrying out a normalizing process for unifying the number of data included in a single beat, and then finding the mean of the blood pressure values at each point in time. Next, the indicator extraction unit 50 determines the correlation between the representative blood pressure waveforms during rest and during activity using a cross-correlation function. Note that the indicator extraction unit 50 carries out the normalizing process for unifying the number of data included in the representative blood pressure waveforms during rest and during activity before calculating the correlation.

The indicator extraction unit 50 calculates the AI value for each beat in the active section, and also averages those values to calculate the average AI value for the active section. Likewise, the indicator extraction unit 50 calculates the AI value for each beat in the resting section, and also averages those values to calculate the average AI value for the resting section. The AI value calculation is a known technique and will therefore not be described here.

In step S109, the indicator extraction unit 50 calculates the overall indicator E on the basis of the indicators T, V, S, and AI. The indicator extraction unit 50 stores the calculated overall indicator E in the storage unit 27 in association with the indicators T, V, S, and AI on which the calculation is based. As described above, the overall indicator E can be calculated as $E = AI + (1 - S) \times \{0.1 \times (1 - T) + 0.2V\}$, but this is merely an example. The following definitions may be given: E increases as the AI value increases; E increases as the effect appearance time T decreases; E increases as the change amount V increases; and E increases as the waveform similarity S decreases.

In step S110, the processing unit 51 obtains the indicators E, T, V, S, and AI stored in the storage unit 27, and generates display screen data for making displays such as those illustrated in Fig. 9A or 9B. The generated display screen data is sent to the output unit 25 and displayed to the user. Alternatively, the generated display data may be sent to another device through the communication unit and displayed.

Advantageous Effects of the Present Embodiment

According to the present embodiment, the load placed on a heart by activity can be evaluated using continuous blood pressure waveform data obtained during free movement. Additionally, a plurality of indicators (sub-indicators) are calculated from different viewpoints with respect to the load on the heart, and an overall indicator that consolidates the sub-indicators is calculated as well, and thus the load on the heart can be evaluated from a variety of viewpoints. Furthermore, the sub-indicators and the overall indicator can be output in a consolidated format, which makes it possible for the user, a doctor, or the like to understand the load on the heart easily and precisely.

Variation

Instead of a doctor inputting the exercise start time and end time, the exercise start time and end time, the active section, and the resting section can be detected on the basis of data measured by the body movement measurement unit 21. Furthermore, the type of exercise can also be determined on the basis of data measured by the body movement measurement unit 21. This makes it possible to evaluate the load on the left ventricle by activity, even in the absence of an assistant such as a doctor.

The configurations in the above-described embodiment and Example are merely specific examples of the present invention, and are not intended to limit the scope of the present invention, which is defined by the appended claims.

INDEX TO THE REFERENCE NUMERALS

1    biological information analyzing device

| 2 | measurement unit |
|---|---|
| 10 | biological information analyzing system |
| 11 | main unit |
| 12 | belt |
| 20 | blood pressure measurement unit |
| 21 | body movement measurement unit |
| 22 | environment measurement unit |
| 23 | control unit |
| 24 | input unit |
| 25 | output unit |
| 26 | communication unit |
| 27 | storage unit |
| 30 | pressure sensor |
| 31 | compression mechanism |
| 300 | pressure detection element |
| 50 | indicator extraction unit |
| 51 | processing unit |

**Claims**

1. A biological information analyzing device (1) comprising:

   an indicator extraction unit (50) that is configured to extract, from blood pressure waveform data measured continuously by a sensor (30) that is wearable on a user's body and which is configured to non-invasively measure a blood pressure waveform for each of a plurality of heartbeats, an indicator expressing a load placed on the user's heart by an activity; and
   a processing unit (51) that is configured to output the indicator extracted by the indicator extraction unit (50), wherein the indicator extraction unit (50) is configured to find the indicator on the basis of a difference between a blood pressure waveform in a resting section corresponding to when the user is in a resting state and a blood pressure waveform in an active section corresponding to when the user is engaged in the activity,
   **characterized in that** the indicator extraction unit (50) is configured to find, on the basis of the difference between the blood pressure waveform in the resting section and the blood pressure waveform in the active section, an effect appearance point that is a time at which an effect of the activity appears as a change in the blood pressure waveform, and is configured to find the indicator on the basis of an amount of time that has passed from the start of the activity to the effect appearance point,
   and **in that** the indicator extraction unit (50) is configured to determine the active section and the resting section on the basis of data expressing an activity start time and data expressing an activity end time for the user or on the basis of measurement data from a second sensor that detects the user's activity.

2. The biological information analyzing device (1) according to claim 1,
   wherein the indicator extraction unit (50) is configured to find the first point, among ejection start points following the start of the activity, where a blood pressure value difference from a representative minimum blood pressure value in the resting section is greater than or equal to a threshold (Th), as the effect appearance point.

3. The biological information analyzing device (1) according to any one of claims 1 to 2,
   wherein the indicator extraction unit (50) is configured to find the indicator on the basis of a difference between a representative minimum blood pressure value in the resting section and a representative minimum blood pressure value in the active section.

4. The biological information analyzing device (1) according to claim 3,
   wherein the representative minimum blood pressure value in the active section is a mean minimum blood pressure value in the active section or a maximum value of the minimum blood pressure value in the active section.

5. The biological information analyzing device (1) according to claim 3 or 4,
   wherein the representative minimum blood pressure value in the resting section is a mean minimum blood pressure value from a predetermined amount of time immediately before the active section.

**6.** The biological information analyzing device (1) according to any one of claims 1 to 5,
wherein the indicator extraction unit (50) is further configured to find an indicator on the basis of a shape similarity between the blood pressure waveform in the resting section and the blood pressure waveform in the active section.

**7.** The biological information analyzing device (1) according to claim 6,
wherein the shape similarity is a correlation between a representative blood pressure waveform in the resting section and a representative blood pressure waveform in the active section.

**8.** The biological information analyzing device (1) according to claim 7,
wherein the representative blood pressure waveform in the active section is a blood pressure waveform of one beat or a mean blood pressure waveform of several beats following an effect appearance point that is a time at which an effect of the activity appears as a change in the blood pressure waveform, and
wherein the representative blood pressure waveform in the resting section is a blood pressure waveform of one beat or a mean blood pressure waveform of several beats immediately before the active section.

**9.** The biological information analyzing device (1) according to any one of claims 1 to 8,
wherein the indicator extraction unit (50) is configured to find a plurality of sub-indicators on the basis of a difference between the blood pressure waveform in the resting section and the blood pressure waveform in the active section, and is configured to calculate the indicator on the basis of the plurality of sub-indicators.

**10.** The biological information analyzing device (1) according to claim 9,
wherein the processing unit (51) is configured to output the indicator as a graph that takes at least one of the sub-indicators as a coordinate axis or outputs at least one of the sub-indicators and the indicator as a table.

**11.** A system (10) comprising:

a sensor (30), which is wearable on a user's body and is configured to non-invasively measure a blood pressure waveform for each of a plurality of heartbeats; and
the biological information analyzing device (1) according to any one of claims 1 to 10, the biological information analyzing device (1) which is configured to analyze biological information using data of the blood pressure waveform measured continuously by the sensor (30).

**12.** A program causing a processor to function as the indicator extraction unit (50) and the processing unit (51) of the biological information analyzing device (1) according to any one of claims 1 to 10.

**13.** A biological information analyzing method comprising:

a step of acquiring, from blood pressure waveform data measured continuously by a sensor (30) wearable on a user's body and capable of non-invasively measuring a blood pressure waveform for each of a plurality of heartbeats, a blood pressure waveform in a resting section corresponding to when the user is in a resting state and a blood pressure waveform in an active section corresponding to when the user is engaged in an activity;
a step of extracting, by an indicator extraction unit (50), an indicator expressing a load placed on the user's heart by the activity on the basis of a difference between the blood pressure waveform in the resting section corresponding to when the user is in a resting state and the blood pressure waveform in the active section corresponding to when the user is engaged in the activity; and
a step of outputting the extracted indicator,
**characterized in that** the indicator extraction unit (50) finds, on the basis of the difference between the blood pressure waveform in the resting section and the blood pressure waveform in the active section, an effect appearance point that is a time at which an effect of the activity appears as a change in the blood pressure waveform, and finds the indicator on the basis of an amount of time that has passed from the start of the activity to the effect appearance point,
and **in that** the indicator extraction unit (50) determines the active section and the resting section on the basis of data expressing an activity start time and data expressing an activity end time for the user or on the basis of measurement data from a second sensor that detects the user's activity.

**Patentansprüche**

1. Vorrichtung (1) zur Analyse biologischer Informationen, umfassend:

   eine Indikatorextraktionseinheit (50), die dafür eingerichtet ist, aus Blutdruckwellenformdaten, die kontinuierlich durch einen Sensor (30) gemessen werden, der am Körper eines Benutzers getragen werden kann und der dafür eingerichtet ist, nicht-invasiv eine Blutdruckwellenform für jeden von mehreren Herzschlägen zu messen, einen Indikator zu extrahieren, der eine Belastung ausdrückt, die durch eine Aktivität auf das Herz des Benutzers ausgeübt wird; und
   eine Verarbeitungseinheit (51), die dafür eingerichtet ist, den durch die Indikatorextraktionseinheit (50) extrahierten Indikator auszugeben,
   wobei die Indikatorextraktionseinheit (50) dafür eingerichtet ist, den Indikator auf der Grundlage einer Differenz zwischen einer Blutdruckwellenform in einem Ruheabschnitt, welcher der Situation entspricht, wenn sich der Benutzer in einem Ruhezustand befindet, und einer Blutdruckwellenform in einem aktiven Abschnitt, welcher der Situation entspricht, wenn der Benutzer mit der Aktivität befasst ist, zu finden,
   **dadurch gekennzeichnet, dass**
   die Indikatorextraktionseinheit (50) dafür eingerichtet ist, auf der Grundlage der Differenz zwischen der Blutdruckwellenform im Ruheabschnitt und der Blutdruckwellenform im aktiven Abschnitt einen Effekterscheinungspunkt zu finden, der ein Zeitpunkt ist, an dem ein Effekt der Aktivität als eine Änderung in der Blutdruckwellenform erscheint, und dafür eingerichtet ist, den Indikator auf der Grundlage einer Zeitspanne zu finden, die vom Beginn der Aktivität bis zu dem Effekterscheinungspunkt vergangen ist, und
   dass die Indikatorextraktionseinheit (50) dafür eingerichtet ist, den aktiven Abschnitt und den Ruheabschnitt auf der Grundlage von Daten, die eine Aktivitätsbeginnzeit ausdrücken, und Daten, die eine Aktivitätsendzeit für den Benutzer ausdrücken, oder auf der Grundlage von Messdaten von einem zweiten Sensor, der die Aktivität des Benutzers detektiert, zu bestimmen.

2. Vorrichtung (1) zur Analyse biologischer Informationen nach Anspruch 1,
   wobei die Indikatorextraktionseinheit (50) dafür eingerichtet ist, den ersten Punkt unter Auswurfbeginnpunkten, die dem Beginn der Aktivität folgen, wo eine Blutdruckwertdifferenz von einem repräsentativen Minimumblutdruckwert in dem Ruheabschnitt mindestens so groß wie eine Schwelle (Th) ist, als den Effekterscheinungspunkt zu finden.

3. Vorrichtung (1) zur Analyse biologischer Informationen nach einem der Ansprüche 1 bis 2,
   wobei die Indikatorextraktionseinheit (50) dafür eingerichtet ist, den Indikator auf der Grundlage einer Differenz zwischen einem repräsentativen Minimumblutdruckwert in dem Ruheabschnitt und einem repräsentativen Minimumblutdruckwert in dem aktiven Abschnitt zu finden.

4. Vorrichtung (1) zur Analyse biologischer Informationen nach Anspruch 3,
   wobei der repräsentative Minimumblutdruckwert in dem aktiven Abschnitt ein mittlerer Minimumblutdruckwert in dem aktiven Abschnitt oder ein Maximalwert des Minimumblutdruckwertes in dem aktiven Abschnitt ist.

5. Vorrichtung (1) zur Analyse biologischer Informationen nach Anspruch 3 oder 4,
   wobei der repräsentative Minimumblutdruckwert in dem Ruheabschnitt ein mittlerer Minimumblutdruckwert aus einer zuvor festgelegten Zeitdauer unmittelbar vor dem aktiven Abschnitt ist.

6. Vorrichtung (1) zur Analyse biologischer Informationen nach einem der Ansprüche 1 bis 5,
   wobei die Indikatorextraktionseinheit (50) des Weiteren dafür eingerichtet ist, einen Indikator auf der Grundlage einer Formähnlichkeit zwischen der Blutdruckwellenform in dem Ruheabschnitt und der Blutdruckwellenform in dem aktiven Abschnitt zu finden.

7. Vorrichtung (1) zur Analyse biologischer Informationen nach Anspruch 6,
   wobei die Formähnlichkeit eine Korrelation zwischen einer repräsentativen Blutdruckwellenform in dem Ruheabschnitt und einer repräsentativen Blutdruckwellenform in dem aktiven Abschnitt ist.

8. Vorrichtung (1) zur Analyse biologischer Informationen nach Anspruch 7,
   wobei die repräsentative Blutdruckwellenform in dem aktiven Abschnitt eine Blutdruckwellenform eines einzelnen Schlages oder eine mittlere Blutdruckwellenform mehrerer Schläge im Anschluss an einen Effekterscheinungspunkt ist, der ein Zeitpunkt ist, an dem ein Effekt der Aktivität als eine Änderung in der Blutdruckwellenform erscheint, und wobei die repräsentative Blutdruckwellenform in dem Ruheabschnitt eine Blutdruckwellenform eines einzelnen Schlages oder eine mittlere Blutdruckwellenform mehrerer Schläge unmittelbar vor dem aktiven Abschnitt ist.

9. Vorrichtung (1) zur Analyse biologischer Informationen nach einem der Ansprüche 1 bis 8, wobei die Indikatorextraktionseinheit (50) dafür eingerichtet ist, mehrere Subindikatoren auf der Grundlage einer Differenz zwischen der Blutdruckwellenform in dem Ruheabschnitt und der Blutdruckwellenform in dem aktiven Abschnitt zu finden, und dafür eingerichtet ist, den Indikator auf der Grundlage der mehreren Subindikatoren berechnet.

10. Vorrichtung (1) zur Analyse biologischer Informationen nach Anspruch 9, wobei die Verarbeitungseinheit (51) dafür eingerichtet ist, den Indikator als ein Diagramm auszugeben, das mindestens einen der Subindikatoren als eine Koordinatenachse nimmt, oder mindestens einen der Subindikatoren und den Indikator als eine Tabelle ausgibt.

11. System(10), umfassend:

   einen Sensor (30), der am Körper eines Benutzers getragen werden kann und dafür eingerichtet ist, nicht-invasiv eine Blutdruckwellenform für jeden von mehreren Herzschlägen zu messen; und
   die Vorrichtung (1) zur Analyse biologischer Informationen nach einem der Ansprüche 1 bis 10, wobei die Vorrichtung (1) zur Analyse biologischer Informationen dafür eingerichtet ist, biologische Informationen unter Verwendung von Daten der Blutdruckwellenform zu analysieren, die kontinuierlich durch den Sensor (30) gemessen wird.

12. Programm, das einen Prozessor veranlasst, als die Indikatorextraktionseinheit (50) und die Verarbeitungseinheit (51) der Vorrichtung (1) zur Analyse biologischer Informationen nach einem der Ansprüche 1 bis 10 zu dienen.

13. Verfahren zur Analyse biologischer Informationen, umfassend:

   einen Schritt des Erfassens, aus Blutdruckwellenformdaten, die kontinuierlich durch einen Sensor (30) gemessen werden, der am Körper eines Benutzers getragen werden kann und in der Lage ist, nicht-invasiv eine Blutdruckwellenform für jeden von mehreren Herzschlägen zu messen, einer Blutdruckwellenform in einem Ruheabschnitt, welcher der Situation entspricht, wenn sich der Benutzer in einem Ruhezustand befindet, und einer Blutdruckwellenform in einem aktiven Abschnitt, welcher der Situation entspricht, wenn der Benutzer einer Aktivität nachgeht;
   einen Schritt des Extrahierens, durch eine Indikatorextraktionseinheit (50), eines Indikators, der eine durch die Aktivität auf das Herz des Benutzers ausgeübte Belastung auf der Grundlage einer Differenz zwischen der Blutdruckwellenform in dem Ruheabschnitt, welcher der Situation entspricht, wenn sich der Benutzer in einem Ruhezustand befindet, und einer Blutdruckwellenform in dem aktiven Abschnitt, welcher der Situation entspricht, wenn der Benutzer mit der Aktivität befasst ist, ausdrückt; und
   einen Schritt des Ausgebens des extrahierten Indikators,
   **dadurch gekennzeichnet, dass**
   die Indikatorextraktionseinheit (50) auf der Grundlage der Differenz zwischen der Blutdruckwellenform im Ruheabschnitt und der Blutdruckwellenform im aktiven Abschnitt einen Effekterscheinungspunkt findet, der ein Zeitpunkt ist, an dem ein Effekt der Aktivität als eine Änderung in der Blutdruckwellenform erscheint, und den Indikator auf der Grundlage einer Zeitspanne findet, die vom Beginn der Aktivität bis zu dem Effekterscheinungspunkt vergangen ist, und
   dass die Indikatorextraktionseinheit (50) den aktiven Abschnitt und den Ruheabschnitt auf der Grundlage von Daten, die eine Aktivitätsbeginnzeit ausdrücken, und Daten, die eine Aktivitätsendzeit für den Benutzer ausdrücken, oder auf der Grundlage von Messdaten von einem zweiten Sensor, der die Aktivität des Benutzers detektiert, bestimmt.

**Revendications**

1. Dispositif d'analyse d'informations biologiques (1) comprenant :

   une unité d'extraction d'indicateur (50) qui est configurée pour extraire, de données de forme d'onde de pression artérielle mesurées en continu par un capteur (30) qui peut être porté sur le corps d'un utilisateur et qui est conçu pour mesurer de façon non effractive une forme d'onde de pression artérielle pour chaque battement de cœur d'une pluralité de battements de cœur, un indicateur exprimant une charge imposée au cœur de l'utilisateur par une activité ; et

une unité de traitement (51) qui est configurée pour délivrer l'indicateur extrait par l'unité d'extraction d'indicateur (50),

dans lequel l'unité d'extraction d'indicateur (50) est configurée pour trouver l'indicateur sur la base d'une différence entre une forme d'onde de pression artérielle dans une section de repos correspondant à un moment où l'utilisateur est à l'état de repos et une forme d'onde de pression artérielle dans une section active correspondant à un moment où l'utilisateur se livre à l'activité,

**caractérisé en ce que** l'unité d'extraction d'indicateur (50) est configurée pour trouver, sur la base de la différence entre la forme d'onde de pression artérielle dans la section de repos et la forme d'onde de pression artérielle dans la section active, un point d'apparition d'effet qui est un instant où un effet de l'activité apparaît comme une modification de la forme d'onde de pression artérielle, et est configurée pour trouver l'indicateur sur la base d'une durée qui s'est écoulée du début de l'activité au point d'apparition d'effet,

et **en ce que** l'unité d'extraction d'indicateur (50) est configurée pour déterminer la section active et la section de repos sur la base de données exprimant un instant de début d'activité et de données exprimant un instant de fin d'activité pour l'utilisateur, ou sur la base de données de mesure provenant d'un second capteur qui détecte l'activité de l'utilisateur.

2. Dispositif d'analyse d'informations biologiques (1) selon la revendication 1,
dans lequel l'unité d'extraction d'indicateur (50) est configurée pour trouver le premier point, parmi des points de début d'éjection faisant suite au début de l'activité, où une différence de valeur de pression artérielle par rapport à une valeur de pression artérielle minimale représentative dans la section de repos est supérieure ou égale à un seuil (Th), en tant que point d'apparition d'effet.

3. Dispositif d'analyse d'informations biologiques (1) selon l'une quelconque des revendications 1 et 2,
dans lequel l'unité d'extraction d'indicateur (50) est configurée pour trouver l'indicateur sur la base d'une différence entre une valeur de pression artérielle minimale représentative dans la section de repos et une valeur de pression artérielle minimale représentative dans la section active.

4. Dispositif d'analyse d'informations biologiques (1) selon la revendication 3,
dans lequel la valeur de pression artérielle minimale représentative dans la section active est une valeur de pression artérielle minimale moyenne dans la section active ou une valeur maximale de la valeur de pression artérielle minimale dans la section active.

5. Dispositif d'analyse d'informations biologiques (1) selon la revendication 3 ou 4,
dans lequel la valeur de pression artérielle minimale représentative dans la section de repos est une valeur de pression artérielle minimale moyenne sur une durée prédéterminée immédiatement avant la section active.

6. Dispositif d'analyse d'informations biologiques (1) selon l'une quelconque des revendications 1 à 5,
dans lequel l'unité d'extraction d'indicateur (50) est configurée en outre pour trouver un indicateur sur la base d'une similitude de forme entre la forme d'onde de pression artérielle dans la section de repos et la forme d'onde de pression artérielle dans la section active.

7. Dispositif d'analyse d'informations biologiques (1) selon la revendication 6,
dans lequel la similitude de forme est une corrélation entre une forme d'onde de pression artérielle représentative dans la section de repos et une forme d'onde de pression artérielle représentative dans la section active.

8. Dispositif d'analyse d'informations biologiques (1) selon la revendication 7,
dans lequel la forme d'onde de pression artérielle représentative dans la section active est une forme d'onde de pression artérielle d'un battement ou une forme d'onde de pression artérielle moyenne de plusieurs battements faisant suite à un point d'apparition d'effet qui est un instant où un effet de l'activité apparaît comme une modification de la forme d'onde de pression artérielle, et

dans lequel la forme d'onde de pression artérielle représentative dans la section de repos est une forme d'onde de pression artérielle d'un battement ou une forme d'onde de pression artérielle moyenne de plusieurs battements immédiatement avant la section active.

9. Dispositif d'analyse d'informations biologiques (1) selon l'une quelconque des revendications 1 à 8,
dans lequel l'unité d'extraction d'indicateur (50) est configurée pour trouver une pluralité de sous-indicateurs sur la base d'une différence entre la forme d'onde de pression artérielle dans la section de repos et la forme d'onde de pression artérielle dans la section active, et est configurée pour calculer l'indicateur sur la base de la pluralité de sous-indicateurs.

10. Dispositif d'analyse d'informations biologiques (1) selon la revendication 9,
dans lequel l'unité de traitement (51) est configurée pour délivrer l'indicateur sous la forme d'un graphique qui a pour axe des coordonnées au moins l'un des sous-indicateurs ou qui délivre au moins l'un des sous-indicateurs et l'indicateur sous la forme d'une table.

11. Système (10) comprenant :

un capteur (30) qui peut être porté sur le corps d'un utilisateur et qui est conçu pour mesurer de façon non effractive une forme d'onde de pression artérielle pour chaque battement de cœur d'une pluralité de battements de cœur; et
le dispositif d'analyse d'informations biologiques (1) selon l'une quelconque des revendications 1 à 10, dispositif d'analyse d'informations biologiques (1) qui est conçu pour analyser des informations biologiques à l'aide de données de la forme d'onde de pression artérielle mesurée en continu par le capteur (30).

12. Programme faisant fonctionner un processeur comme l'unité d'extraction d'indicateur (50) et l'unité de traitement (51) du dispositif d'analyse d'informations biologiques (1) selon l'une quelconque des revendications 1 à 10.

13. Procédé d'analyse d'informations biologiques comprenant :

une étape consistant à acquérir, à partir de données de forme d'onde de pression artérielle mesurées en continu par un capteur (30) pouvant être porté sur le corps d'un utilisateur et étant apte à mesurer de façon non effractive une forme d'onde de pression artérielle pour chaque battement de cœur d'une pluralité de battements de cœur, une forme d'onde de pression artérielle dans une section de repos correspondant à un moment où l'utilisateur est à l'état de repos et une forme d'onde de pression artérielle dans une section active correspondant à un moment où l'utilisateur se livre à une activité,
une étape consistant à extraire, au moyen d'une unité d'extraction d'indicateur (50), un indicateur exprimant une charge imposée au cœur de l'utilisateur par l'activité, sur la base d'une différence entre la forme d'onde de pression artérielle dans la section de repos correspondant à un moment où l'utilisateur est à l'état de repos et la forme d'onde de pression artérielle dans la section active correspondant à un moment où l'utilisateur se livre à l'activité ; et
une étape consistant à délivrer l'indicateur extrait,
**caractérisé en ce que** l'unité d'extraction d'indicateur (50) trouve, sur la base de la différence entre la forme d'onde de pression artérielle dans la section de repos et la forme d'onde de pression artérielle dans la section active, un point d'apparition d'effet qui est un instant où un effet de l'activité apparaît comme une modification de la forme d'onde de pression artérielle, et trouve l'indicateur sur la base d'une durée qui s'est écoulée du début de l'activité au point d'apparition d'effet,
et **en ce que** l'unité d'extraction d'indicateur (50) détermine la section active et la section de repos sur la base de données exprimant un instant de début d'activité et de données exprimant un instant de fin d'activité pour l'utilisateur, ou sur la base de données de mesure provenant d'un second capteur qui détecte l'activité de l'utilisateur.

FIG. 1

FIG. 2

EP 3 427 649 B1

FIG. 3

FIG. 4

FIG. 5

FIG. 6

FIG. 7

FIG. 8

FIG. 9A

| | | ACTIVITY | | | |
|---|---|---|---|---|---|
| | | RESTING | SPREAD-ING | ROLLING OVER | WALKING |
| INDICATOR | AI | 0.3 | 0.3 | 0.32 | 0.32 |
| | EFFECT APPREARANCE TIME T(s) | – | 0.1 | 0.07 | 0.2 |
| | EFFECT AMOUNT V(mmHg) | – | 0.3 | 0.8 | 4.1 |
| | WAVEFORM SIMILARITY S | – | 0.8 | 0.72 | 0.63 |
| | OVERALL INDICATOR E | 0.3 | 0.33 | 0.391 | 0.653 |

FIG. 9B

EFFECT AMOUNT V

WALKING 1601

SPREADING LEGS

1603

ROLLING OVER 1602

EFFECT APPEARANCE TIME T

FIG. 10

```
                    ┌─────────────────┐
                    │      START      │
                    └─────────────────┘
                             │
                             ▼
        ┌────────────────────────────────────────┐
        │  START MEASURING BLOOD PRESSURE         │  S101
        │  WAVEFORM DATA                          │
        └────────────────────────────────────────┘
                             │
                             ▼
        ┌────────────────────────────────────────┐
        │  START EXERCISE                         │  S102
        │  (ACQUIRE START TIME)                   │
        └────────────────────────────────────────┘
                             │
                             ▼
        ┌────────────────────────────────────────┐
        │  END EXERCISE                           │  S103
        │  (ACQUIRE END TIME)                     │
        └────────────────────────────────────────┘
                             │
                             ▼
        ┌────────────────────────────────────────┐
        │  END MEASURING BLOOD PRESSURE           │  S104
        │  WAVEFORM DATA                          │
        └────────────────────────────────────────┘
                             │
                             ▼
        ┌────────────────────────────────────────┐
        │  DIVIDE BLOOD PRESSURE WAVEFORM DATA    │  S105
        │  INTO UNITS OF BEATS                    │
        └────────────────────────────────────────┘
                             │
                             ▼
        ┌────────────────────────────────────────┐
        │  ACQUIRE MINIMUM BLOOD PRESSURE VALUE   │  S106
        │  DURING REST                            │
        └────────────────────────────────────────┘
                             │
                             ▼
        ┌────────────────────────────────────────┐
        │  DETECT EFFECT APPEARANCE POINT         │  S107
        └────────────────────────────────────────┘
                             │
                             ▼
        ┌────────────────────────────────────────┐
        │  CALCULATE EXERCISE LOAD                │  S108
        │  INDICATORS (T, V, S)                   │
        └────────────────────────────────────────┘
                             │
                             ▼
        ┌────────────────────────────────────────┐
        │  CALCULATE OVERALL INDICATOR ON BASIS   │  S109
        │  OF EXERCISE LOAD INDICATORS (T, V, S)  │
        └────────────────────────────────────────┘
                             │
                             ▼
        ┌────────────────────────────────────────┐
        │  OUPUT OVERALL INDICATOR                │  S110
        └────────────────────────────────────────┘
                             │
                             ▼
                    ┌─────────────────┐
                    │      END        │
                    └─────────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008061824 A
- JP 2005532111 A
- EP 1334693 A1
- JP 3820719 B
- JP 2016082069 A
- JP 2016087003 A